# DEMANDE DE BREVET EUROPEEN

(11) **EP 3 032 553 A1**
(43) Date de publication de la demande: **15.06.2016**
(21) Numéro de dépôt: 15197758.4
(22) Date de dépôt: 03.12.2015
(51) Int. Cl.: H01F 27/14

(54) **DISPOSITIF DE DIAGNOSTIC POUR TRANSFORMATEUR ELECTRIQUE IMMERGE ET TRANSFORMATEUR ELECTRIQUE COMPRENANT UN TEL DISPOSITIF**

(30) Priorité: 10.12.2014 FR 1462190
(71) Demandeur: SNCF MOBILITES, 93200 Saint-Denis (FR)
(72) Inventeur: CHAMARET, André-Philippe, 72000 Le Mans (FR)
(74) Mandataire: Pontet Allano & Associes

(57) **Abrégé**

La présente invention concerne un dispositif de diagnostic (100) pour transformateur électrique immergé (130), comprenant :
- au moins un module de mesure pour mesurer au moins une grandeur physique relative au diélectrique liquide (170) dudit transformateur immergé (130), et
- au moins un circuit de prélèvement, prévu pour être relié audit transformateur immergé (130) et, prélever et acheminer au moins un échantillon dudit diélectrique liquide (170) vers l'au moins un module de mesure.

Elle concerne également un transformateur électrique immergé comprenant un tel dispositif de diagnostic.

## Description

### Domaine technique

La présente invention concerne un dispositif de diagnostic pour transformateur électrique immergé dans un liquide isolant.

La présente invention se situe dans le domaine des transformateurs électriques immergés dans un liquide isolant ou un diélectrique liquide.

### Etat de la technique antérieure

De manière connue, les transformateurs immergés sont constitués d'au moins une cuve contenant notamment les parties électriques du transformateur ainsi qu'un isolant liquide afin non seulement d'isoler les conducteurs électriques entre eux et par rapport à la masse, mais aussi de favoriser les échanges thermiques entre les enroulements électriques et un système de réfrigération.

L'isolant liquide joue ainsi un rôle fondamental dans le bon fonctionnement du transformateur électrique immergé et il est important que ses caractéristiques intrinsèques soient conservées au cours du temps. Or, l'efficacité du liquide isolant diminue en vieillissant du fait de l'action conjointe de la température, de l'air et de l'humidité.

Actuellement, l'état de l'isolant liquide est surveillé par des analyses périodiques, par exemple tous les 18 ou 36 mois, par prélèvements manuels des transformateurs immergés sur les installations immobilisées pour l'occasion. Dans le domaine ferroviaire, ces opérations sont réalisées dans des ateliers de maintenance vers lesquels les transformateurs prélevés sont transportés.

Or, ce procédé de surveillance présente plusieurs inconvénients. Tout d'abord, il ne permet pas un contrôle en temps réel de l'état du diélectrique liquide contenu dans le transformateur et il existe ainsi une période durant laquelle l'état du liquide isolant dans le transformateur peut se détériorer sans contrôle et générer des avaries importantes pouvant réduire les performances du transformateur voire stopper le fonctionnement par exemple de la chaîne de traction dans le cas d'un véhicule ferroviaire.

Par ailleurs, les opérations de prélèvement et de transport d'un transformateur sont longues et fastidieuses.

En outre, lors de chaque analyse périodique, le transformateur concerné est immobilisé, ce qui entraine une perte d'exploitation de l'appareil dans lequel le transformateur est utilisé.

Enfin, les opérations de prélèvement, de transport et d'analyse sont dangereuses pour les opérateurs manipulant le transformateur et/ou le liquide isolant et sensibles pour le matériel. Elles nécessitent un véritable savoir-faire de sorte qu'elles ne peuvent pas être réalisées par tous les opérateurs.

La présente invention a pour objet de répondre au moins en grande partie aux problèmes précédents et de conduire en outre à d'autres avantages.

Un but de l'invention est de réduire les risques d'avarie d'un transformateur électrique immergé.

Un autre but de la présente invention est de permettre de contrôler en temps réel l'état du diélectrique liquide d'un transformateur électrique immergé.

Il est aussi un autre but de la présente invention de réduire, le coût, les risques et/ou le temps de maintenance d'un transformateur électrique immergé.

Encore un autre but de la présente invention est de réduire le temps d'immobilisation du matériel fonctionnant avec un tel transformateur.

### Exposé de l'invention

L'invention permet d'atteindre au moins l'un des objectifs précités avec un dispositif de diagnostic pour transformateur électrique immergé comprenant :
- au moins un module de mesure pour mesurer au moins une grandeur physique relative au diélectrique liquide dudit transformateur immergé ; et
- au moins un circuit de prélèvement, prévu pour être relié audit transformateur immergé et, prélever et acheminer au moins un échantillon dudit diélectrique liquide vers l'au moins un module de mesure.

Il est ainsi possible grâce à la présente invention de mesurer des informations pertinentes sur l'état du liquide diélectrique d'un transformateur électrique immergé et d'en analyser différentes propriétés physiques afin de vérifier le bon état de fonctionnement du transformateur électrique immergé correspondant, sans avoir à prélever le transformateur électrique lui-même.

Le dispositif selon l'invention permet de réaliser les tests de vérification de manière plus automatique, voire continue, et plus efficace car le diélectrique liquide est prélevé sans intervention humaine pour être mesuré par le module de mesure.

De plus, le dispositif selon l'invention permet de réaliser la maintenance des transformateurs électriques immergés de manière plus optimale et en réduisant les coûts, sans nécessiter de savoir-faire spécifique, car il n'est plus nécessaire d'immobiliser le matériel fonctionnant avec le transformateur, par exemple un véhicule ferroviaire, ni de prélever et de transporter le transformateur.

Avantageusement, le circuit de prélèvement du dispositif de diagnostic selon l'invention peut comprendre un premier conduit, dit de prélèvement, apte à prélever au moins un échantillon du diélectrique liquide du transformateur électrique immergé et un second conduit, dit d'évacuation, pour évacuer ledit au moins un échantillon hors du module de mesure.

Selon un premier mode de réalisation, le conduit d'évacuation peut être un conduit de purge de l'au moins un échantillon prélevé dans le transformateur électrique immergé, en vue de sa mise au rebut. L'au moins un échantillon est alors sorti de manière définitive du transformateur et n'est plus utilisé dans le transformateur. Ce mode de réalisation permet notamment de ne pas polluer le diélectrique liquide présent dans le transformateur électrique lorsque des mesures qui modifient ses propriétés physiques sont réalisées par le module de mesure.

Selon un deuxième mode de réalisation, le conduit d'évacuation peut être relié au transformateur de sorte que l'échantillon prélevé est réinjecté vers ledit transformateur. Ainsi, le niveau de l'isolant liquide présent dans le transformateur électrique n'est pas modifié par les mesures réalisées.

Selon une autre caractéristique avantageuse, le conduit de prélèvement du dispositif de diagnostic selon l'invention peut comprendre au moins une vanne programmable d'entrée et/ou le conduit d'évacuation peut comprendre au moins une vanne programmable de sortie. Ainsi, le prélèvement de l'au moins un échantillon de diélectrique liquide dans le transformateur et/ou l'évacuation dudit échantillon du module de mesure, est (sont) automatisé(s). Il est ainsi possible de minimiser, voire supprimer, les interventions humaines sur de telles mesures ainsi que les conséquences d'une telle intervention : risques de contamination, main d'oeuvre, coût, etc.

Selon une autre caractéristique avantageuse, le module de mesure du dispositif de diagnostic selon l'invention peut comprendre :
- au moins un moyen de mesure de la rigidité du diélectrique liquide ; et/ou
- au moins un moyen de mesure de l'acidité du diélectrique liquide ; et/ou
- au moins un moyen pour mesurer les gaz dissous dans le diélectrique liquide ; et/ou
- au moins un moyen pour mesurer la teneur en eau du diélectrique liquide ; et/ou
- au moins un moyen pour mesurer le niveau de diélectrique liquide présent dans le transformateur électrique immergé.

D'une manière plus générale, le module de mesure peut comprendre tout autre moyen de mesure permettant de caractériser des paramètres physiques, chimiques, électriques du diélectrique liquide et représentatifs de son état vis-à-vis de son rôle dans le fonctionnement du transformateur électrique. Plus particulièrement, toute mesure permettant de déterminer un paramètre qui influence la capacité du diélectrique liquide à jouer un rôle d'isolant électrique et/ou de conducteur thermique peut faire l'objet d'une mesure complémentaire par le dispositif de diagnostic selon l'invention.

Les différentes mesures réalisées par le module de mesure peuvent être réalisées de manière continue ou intermittente, et de manière synchronisée ou asynchrone.

Préférentiellement, le dispositif selon l'invention peut comprendre en outre un module d'analyse pour analyser et/ou interpréter l'au moins une mesure réalisée par le module de mesure par rapport à au moins une valeur de référence préalablement déterminée.

Plus particulièrement, le module d'analyse peut détecter par exemple certaines situations prédéfinies et déclencher le cas échéant des opérations de maintenance prédéfinies. Il peut aussi, cumulativement ou alternativement, détecter des situations non prévues afin de signaler une situation d'urgence pour le transformateur et/ou le matériel roulant sur lequel ce dernier est embarqué.

Avantageusement, le dispositif selon l'invention peut comprendre un module de communication pour transmettre au moins une donnée mesurée et/ou analysée à un système d'information :
- d'un véhicule dans lequel se trouve le transformateur électrique immergé auquel est associé ledit dispositif de diagnostic, et/ou
- local au transformateur électrique immergé auquel est associé ledit dispositif de diagnostic ; et/ou
- distant dudit transformateur électrique immergé auquel est associé ledit dispositif de diagnostic.

La communication entre ledit module de communication et ledit système d'information peut se faire par tous moyens connus de l'homme du métier, filaires ou sans fil.

Selon une autre caractéristique avantageuse, le dispositif de diagnostic selon l'invention peut en outre comprendre au moins un moyen pour faire circuler l'au moins un échantillon de diélectrique liquide dans ledit dispositif, et en particulier dans le circuit de prélèvement, et encore plus particulièrement dans le conduit de prélèvement, puis dans le module de mesure et ensuite dans le conduit d'extraction

Un tel moyen pour faire circuler le diélectrique liquide peut comprendre au moins une pompe disposée dans/sur/au niveau :
- du conduit de prélèvement, et/ou
- du conduit d'évacuation, et/ou
- du module de mesure.

Alternativement, le dispositif de diagnostic selon l'invention peut ne pas comprendre de moyen pour faire circuler le diélectrique liquide. Dans ce cas, le diélectrique liquide peut être prélevé par exemple par capillarité ou par gravité d'une part, ou d'autre part grâce à des mouvements naturels du diélectrique liquide dans le transformateur (convection thermique...) ou induits par tout élément du transformateur, comme par exemple une pompe dans un circuit de refroidissement.

Suivant un autre aspect de l'invention, il est proposé un transformateur électrique immergé muni d'un dispositif de diagnostic selon l'invention. Ainsi, il est possible de contrôler en temps réel l'état du diélectrique liquide d'un transformateur électrique immergé car ce mode de réalisation permet d'embarquer le dispositif de diagnostic sur le transformateur lui-même.

Selon un mode particulier de réalisation, le circuit de prélèvement du dispositif de diagnostic peut être relié à une cuve dudit transformateur immergé.

Selon un mode de réalisation alternatif, lorsque le transformateur électrique comprend, ou est associé à, un circuit de refroidissement du diélectrique liquide dudit transformateur immergé, alors le circuit de prélèvement du dispositif de diagnostic peut être connecté/relié :
- en dérivation sur :
   ▪ ledit circuit de refroidissement du diélectrique liquide dudit transformateur immergé, ou
   ▪ un élément quelconque dudit circuit de refroidissement, et en particulier une pompe à huile, un échangeur thermique, etc. ;
- en série dans ledit circuit de refroidissement, de sorte que l'ensemble de l'isolant liquide circulant dans ledit circuit circule dans ledit dispositif de diagnostic.

Suivant un autre aspect de l'invention, il est proposé un procédé de diagnostic du diélectrique liquide contenu dans un transformateur électrique immergé selon l'invention et comprenant les étapes suivantes :
- prélèvement du diélectrique liquide contenu dans le transformateur électrique immergé,
- mesure d'au moins une grandeur physique caractérisant le diélectrique liquide par le module de mesure,
- analyse de l'au moins une mesure réalisée par le module de mesure, et
- transfert de l'au moins une mesure et/ou au moins un résultat d'analyse par le module de communication.

Suivant un mode particulier de réalisation, l'analyse de l'au moins une mesure réalisée par le module de mesure peut être exécutée au fur et à mesure de l'acquisition de ladite au moins une mesure.

Préférentiellement, l'étape de prélèvement du diélectrique liquide contenu dans le transformateur électrique immergé peut comprendre les étapes suivantes :
- ouverture de la vanne programmable d'entrée,
- remplissage du diélectrique liquide dans le module de mesure et
- fermeture de la vanne programmable d'entrée.

Avantageusement, l'étape de mesure d'au moins une grandeur physique caractérisant le diélectrique liquide peut comprendre au moins une des étapes suivantes :
- mesure de l'acidité du diélectrique liquide,
- mesure de la rigidité du diélectrique liquide,
- mesure de la teneur en eau du diélectrique liquide,
- mesure des gaz dissous présents dans le diélectrique liquide, et/ou
- mesure du niveau de diélectrique liquide.

De manière préférentielle, l'étape d'analyse de l'au moins une mesure peut comprendre les étapes suivantes :
- comparaison de l'au moins une mesure avec au moins une valeur de référence, l'au moins un résultat de ladite comparaison formant au moins un résultat d'analyse,
- génération d'au moins un message comprenant une donnée relative audit résultat d'analyse, et
- enregistrement de l'au moins une mesure et/ou de l'au moins un résultat d'analyse.

### Description des figures et des modes de réalisation

D'autres caractéristiques et avantages de l'invention apparaîtront encore au travers de la description qui suit d'une part, et de plusieurs exemples de réalisation donnés à titre indicatif et non limitatif en référence aux dessins schématiques annexés d'autre part, sur lesquels :
- la FIGURE 1 illustre un premier mode de réalisation du transformateur électrique immergé objet de l'invention, dans lequel le dispositif de diagnostic est intégré audit transformateur électrique,
- la FIGURE 2 illustre un second mode de réalisation du transformateur électrique immergé objet de l'invention, dans lequel le dispositif de diagnostic est monté en dérivation dudit transformateur électrique immergé,
- la FIGURE 3 illustre un troisième mode de réalisation du transformateur électrique immergé objet de l'invention, comprenant un dispositif de diagnostic en dérivation et pour lequel le diélectrique liquide n'est pas réintroduit dans le transformateur électrique,

- la FIGURE 4 illustre de manière détaillée un exemple de réalisation d'un dispositif de diagnostic selon l'invention, dans le second mode de réalisation de l'invention,
- la FIGURE 5 illustre un exemple de fonctionnement du dispositif de diagnostic réalisé par l'invention.

Dans la suite, les équipements constitutifs des transformateurs électriques immergés - biens connus de l'homme du métier - ne sont pas représentés sur les figures. Il s'agit par exemple de l'indicateur de circulation d'huile, de la soupape de sécurité, des sondes de températures, des bornes ou encore de la cuve, des enroulements électriques et des circuits magnétiques de la partie active du transformateur.

Les modes de réalisation qui seront décrits dans la suite ne sont nullement limitatifs ; on pourra notamment imaginer des variantes de l'invention ne comprenant qu'une sélection de caractéristiques décrites par la suite isolées des autres caractéristiques décrites, si cette sélection de caractéristiques est suffisante pour conférer un avantage technique ou pour différencier l'invention par rapport à l'état de la technique antérieur. Cette sélection comprend au moins une caractéristique de préférence fonctionnelle sans détails structurels, ou avec seulement une partie des détails structurels si cette partie uniquement est suffisante pour conférer un avantage technique ou pour différencier l'invention par rapport à l'état de la technique antérieur.

En particulier, toutes les variantes et tous les modes de réalisation décrits sont combinables entre eux si rien ne s'oppose à cette combinaison sur le plan technique.

Sur les figures, les éléments communs à plusieurs figures conservent la même référence.

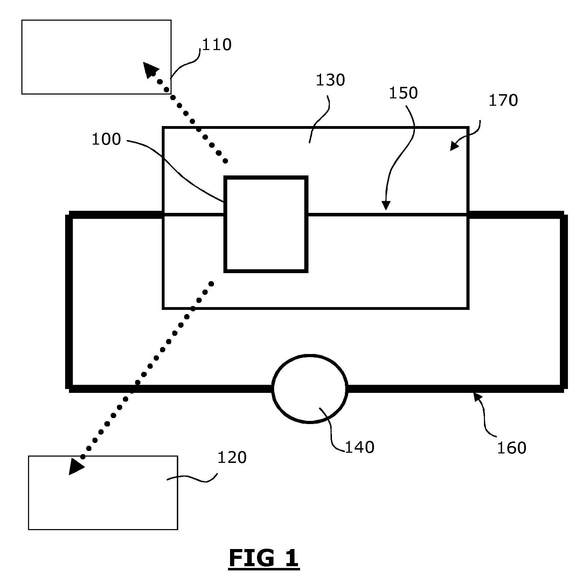

La FIGURE 1 illustre un premier mode de réalisation d'un transformateur immergé selon l'invention, dans lequel le dispositif de diagnostic est intégré audit transformateur électrique.

Le transformateur électrique 130 se compose d'au moins une cuve comprenant les parties actives dudit transformateur, non représentées ici, et baignant dans un diélectrique liquide 170. Afin de faciliter la dissipation thermique du diélectrique liquide 170, un circuit de refroidissement 160 permet, à l'aide d'une pompe 140, de faire circuler le diélectrique liquide 170 d'une part à l'intérieur de la cuve du transformateur électrique 130 et d'autre part dans le circuit de refroidissement 160 afin par exemple d'acheminer ledit diélectrique liquide 170 vers un échangeur thermique (non représenté).

Le dispositif de diagnostic 100 est installé à proximité du transformateur électrique immergé 130 ou à l'intérieur de l'au moins une cuve. Il est relié audit transformateur électrique par l'intermédiaire d'un circuit de prélèvement 150 du diélectrique liquide 170 contenu dans la cuve d'un transformateur électrique immergé 130. Le circuit de prélèvement 150 peut ainsi prélever une petite quantité de liquide diélectrique 170 pour réaliser lesdites mesures ou prélever l'intégralité dudit diélectrique liquide 170 contenu dans l'au moins une cuve. Une fois les mesures réalisées, l'échantillon peut être réinjecté dans la cuve du transformateur. Dans ce premier mode de réalisation, le dispositif de diagnostic 100 est indépendant du circuit de refroidissement 160 du transformateur.

Le dispositif de diagnostic 100 est adapté pour réaliser au moins une mesure d'au moins un paramètre représentatif de l'état du diélectrique liquide 170, et plus particulièrement de ses propriétés de conducteur thermique et d'isolant électrique. Le dispositif de diagnostic 100 réalise également au moins une mesure de certains paramètres du diélectrique liquide 170, et/ou leur analyse vis-à-vis d'au moins une référence et/ou la transmission de ces résultats de mesures et/ou d'analyse par le biais d'un système de communication. Ces résultats sont communiqués alternativement ou conjointement au système d'information 110 du matériel roulant sur lequel le transformateur électrique immergé est embarqué et/ou à un système de télémaintenance 120. Cette communication peut se faire soit de manière filaire ou de manière non filaire, selon n'importe quel moyen connu de l'homme du métier.

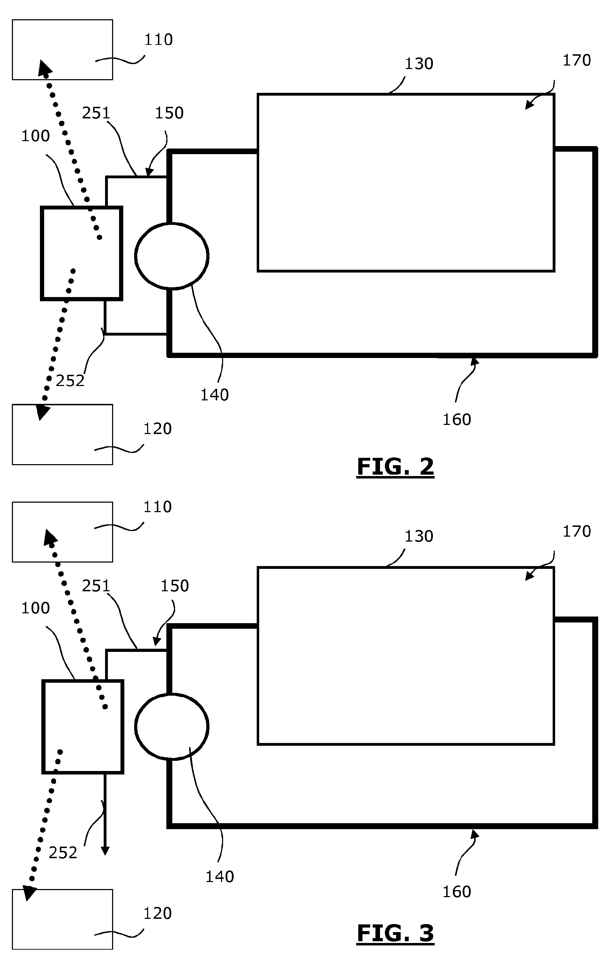

La FIGURE 2 illustre un second mode de réalisation d'un transformateur immergé selon l'invention. A la différence du premier mode de réalisation représenté à la FIGURE 1, le dispositif de diagnostic 100 est installé en dérivation du circuit de refroidissement 160 d'un transformateur électrique immergé 130 - ici au niveau d'une pompe 140 - par l'intermédiaire d'un circuit de prélèvement 150 du diélectrique liquide 170.

Un premier conduit dit de prélèvement 251 relie le circuit de prélèvement 150 au circuit de refroidissement 160 du côté d'un premier côté de la pompe 140, et un second conduit dit d'évacuation 252 relie le circuit de prélèvement 150 au circuit de refroidissement 160 du côté d'un second côté de la pompe 140.

La FIGURE 3 illustre un troisième mode de réalisation d'un transformateur immergé selon l'invention, comparable au second mode de réalisation illustré dans la FIGURE 2, mais dans lequel le conduit d'évacuation 252 n'est pas relié au transformateur 130 : il permet de purger le dispositif de diagnostic 100 de l'au moins un échantillon prélevé de diélectrique liquide 170. Cette alternative permet ainsi de ne pas polluer le transformateur électrique immergé 130 avec le diélectrique liquide 170 sur lequel les mesures ont été réalisées, dans le cas où lesdites mesures modifieraient les caractéristiques physiques et/ou électriques du diélectrique liquide.

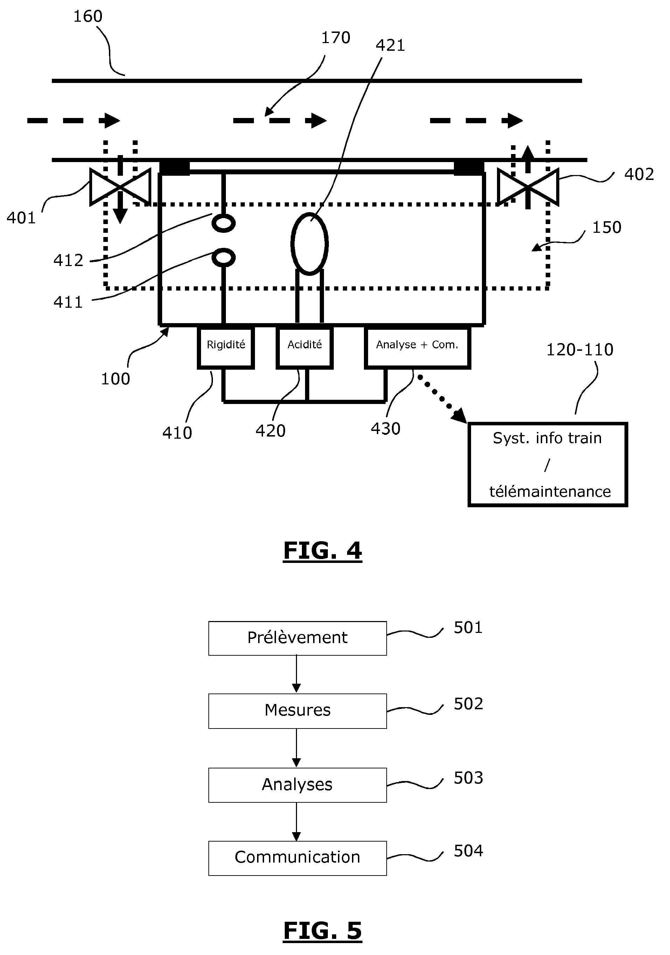

La FIGURE 4 illustre un exemple non limitatif d'un dispositif de diagnostic dans le cadre du second mode de réalisation représenté en FIGURE 2.

Le dispositif de diagnostic 100 est monté en dérivation du circuit de refroidissement 160 d'un transformateur électrique immergé 130. Le diélectrique liquide 170 circule librement et une portion est prélevée par l'intermédiaire d'une vanne d'entrée 401, qui permet au diélectrique liquide de pénétrer dans le circuit de prélèvement 150 et d'être dirigé vers le module de diagnostic 100. Selon un mode de réalisation particulier, tout le volume de diélectrique liquide 170 circulant dans le circuit de refroidissement 160 du transformateur électrique 130 peut être orienté vers dispositif de diagnostic 100.

Dans l'exemple illustré sur la FIGURE 4, le dispositif de diagnostic 100 se compose :
- d'un premier moyen de mesure 410 qui permet de mesurer la rigidité du diélectrique liquide 170 contenu dans le circuit de prélèvement 150 afin de caractériser la capacité d'isolation électrique dudit diélectrique liquide 450, en mesurant la valeur maximale du champ électrique appliqué entre une première borne 411 et une deuxième borne 412 avant le déclenchement d'un arc électrique entre elles. A cet effet, les bornes 411 et 412 sont au moins partiellement noyées dans le diélectrique liquide 170 circulant dans le dispositif de mesure 100 ;
- d'un deuxième moyen de mesure 420 qui permet de mesurer l'acidité du diélectrique liquide 170 contenu dans le circuit de prélèvement 150, par l'intermédiaire d'une électrode de mesure 421 immergée dans le diélectrique liquide 170. Ces mesures peuvent être réalisées de manière optique par exemple, ou selon tout autre moyen connu par l'homme du métier ;
- d'un module d'analyse 430 qui permet en outre d'enregistrer les données mesurées, de les analyser vis-à-vis de certaines valeurs de références et/ou de générer des messages d'interprétation en fonction des résultats d'analyse. Les résultats d'analyse et/ou les messages d'interprétation peuvent aussi être enregistrés sur le module d'analyse. Le module d'analyse peut se composer d'un moyen de mémorisation et d'un processeur afin de réaliser les traitements des données.

Le dispositif de diagnostic 100 comprend en outre un module de communication apte à communiquer avec le système d'information du matériel roulant et/ou de télémaintenance. Le module de communication peut être filaire ou non filaire selon tout moyen connu.

Dans l'exemple illustré sur la FIGURE 4, le dispositif de diagnostic 100 réalise au moins une mesure sur au moins un échantillon de diélectrique liquide prélevé dans le circuit de refroidissement 160 du transformateur électrique immergé 130 et envoyé dans un circuit de prélèvement 150.

Pour ce faire, et comme expliqué précédemment, une vanne d'entrée 401 permet de prélever un échantillon de diélectrique liquide 170 dans le circuit de refroidissement 160 du diélectrique liquide ou dans tout autre circuit contenu dans la cuve du transformateur électrique 130. Le diélectrique liquide est envoyé dans le circuit de prélèvement 150 puis dans le dispositif de diagnostic 100 et une vanne de sortie 402 permet au diélectrique liquide de retourner dans le circuit de refroidissement 160 du transformateur électrique immergé 130.

Selon un mode de réalisation particulier, les vannes d'entrée 401 et de sortie 402 peuvent être programmable et/ou fonctionner de manière synchrone ou asynchrone.

La FIGURE 5 illustre le procédé de diagnostic du diélectrique liquide d'un transformateur électrique immergé selon l'invention.
Il se décompose en quatre étapes :
- une première étape 501 consiste en le prélèvement d'un échantillon de diélectrique liquide 170 dans un transformateur électrique immergé 130. L'objectif est de faire entrer tout ou partie du diélectrique liquide 170 dans le circuit de prélèvement 160 afin de réaliser les différentes mesures et analyses à l'aide du dispositif de diagnostic 100 selon l'invention. Selon un mode particulier de réalisation, cette première étape 501 peut être réalisée par l'ouverture - éventuellement synchronisée - des vannes d'entrée 401 et/ou de sortie 402.
- La seconde étape 502 consiste à faire au moins une mesures à l'aide des moyens de mesure embarqués dans le dispositif de diagnostic 100 selon l'invention.
- La troisième étape 503 consiste à analyser les mesures réalisées à l'aide du module d'analyse. Plus particulièrement, l'au moins une donnée mesurée peut être traitée afin d'être plus facilement exploitable et/ou comparée à au moins un abaque ou à au moins une valeur de référence caractéristique de certains états du diélectrique liquide. L'objectif est d'analyser les données brutes mesurées afin de définir par exemple un critère rendant compte de la qualité du diélectrique liquide mesurée au regard d'au moins un paramètre donné, notamment électrique. Il est possible de définir plusieurs paramètres d'analyse pour un seul moyen de mesure. L'objectif est aussi de mettre en forme au moins un signal d'information qui rend compte de ces mesures et qui peut être transmis et exploité par l'exploitant du dispositif de diagnostic 100. Les données mesurées, analyses et messages d'information peuvent être enregistrés - éventuellement partiellement - durant cette troisième étape.
- La quatrième étape 504 est une étape de communication de l'au moins une donnée et/ou analyse et/ou message informatif généré par le dispositif de diagnostic 100 selon l'invention. L'au moins une donnée est transmise directement au système d'information 110 du matériel roulant sur lequel est embarqué le dispositif de diagnostic 100 et/ou par un système de télémaintenance 120 afin de créer un signalement pour préparer une opération de maintenance adaptée à la défaillance mesurée par le dispositif de diagnostic 100.

Bien sûr, l'invention n'est pas limitée aux exemples qui viennent d'être décrits et de nombreux aménagements peuvent être apportés à ces exemples sans sortir du cadre de l'invention.

## Revendications

1. Dispositif de diagnostic (100) pour transformateur électrique immergé (130), ledit dispositif étant **caractérisé en ce qu'**il comprend :
- au moins un module de mesure pour mesurer au moins une grandeur physique relative au diélectrique liquide (170) dudit transformateur immergé (130) ; et
- au moins un circuit de prélèvement (150), prévu pour être relié audit transformateur immergé (130) et, prélever et acheminer au moins un échantillon dudit diélectrique liquide (170) vers l'au moins un module de mesure ;
ledit module de mesure réalisant une mesure directement sur ledit diélectrique liquide prélevé.

2. Dispositif (100) selon la revendication précédente, **caractérisé en ce que** le circuit de prélèvement (150) comprend un premier conduit, dit de prélèvement (251), apte à prélever au moins un échantillon du diélectrique liquide (170) du transformateur électrique immergé (130) et un second conduit, dit d'évacuation (252), pour évacuer ledit au moins un échantillon hors dudit module de mesure.

3. Dispositif (100) selon la revendication précédente, **caractérisé en ce que** le conduit d'évacuation (252) est un conduit de purge de l'au moins un échantillon en vue d'une mise au rebut.

4. Dispositif (100) selon la revendication 2, **caractérisé en ce que** le conduit d'évacuation (252) est relié au transformateur électrique immergé (130) de sorte que l'échantillon prélevé est réinjecté vers ledit transformateur.

5. Dispositif (100) selon l'une quelconque des revendications 2 à 4, **caractérisé en ce que** le conduit de prélèvement (251) comprend au moins une vanne programmable d'entrée (401) et/ou le conduit d'évacuation (252) comprend une vanne programmable de sortie (402).

6. Dispositif (100) selon l'une quelconque des revendications précédentes, **caractérisé en ce que** le module de mesure comprend :
- au moins un moyen de mesure de la rigidité du diélectrique liquide (170) ; et/ou
- au moins un moyen de mesure de l'acidité du diélectrique liquide (170) ; et/ou
- au moins un moyen pour mesurer les gaz dissous dans le diélectrique liquide (170) ; et/ou
- au moins un moyen pour mesurer la teneur en eau du diélectrique liquide (170) ; et/ou
- au moins un moyen pour mesurer le niveau de diélectrique liquide (170) présent dans le transformateur électrique immergé (130).

7. Dispositif (100) selon l'une quelconque des revendications précédentes, **caractérisé en ce qu'**il comprend en outre un module d'analyse (430) pour analyser et/ou interpréter au moins une mesure réalisée par le module de mesure par rapport à au moins une valeur de référence prédéterminée.

8. Dispositif (100) selon l'une quelconque des revendications précédentes, **caractérisé en ce qu'**il comprend en outre un module de communication pour transmettre de manière filaire ou sans fil l'au moins une donnée mesurée et/ou analysée vers un système d'information :
- d'un véhicule dans lequel se trouve le transformateur électrique immergé auquel est associé ledit dispositif de diagnostic, et/ou
- local au transformateur électrique immergé auquel est associé ledit dispositif de diagnostic ; et/ou
- distant dudit transformateur électrique immergé auquel est associé ledit dispositif de diagnostic.

9. Dispositif (100) selon l'une quelconque des revendications précédentes, **caractérisé en ce qu'**il comprend en outre au moins un moyen pour faire circuler l'au moins un échantillon de diélectrique liquide dans ledit dispositif (100).

10. Transformateur électrique immergé comprenant un dispositif de diagnostic (100) selon l'une quelconque des revendications précédentes.

11. Transformateur électrique immergé selon la revendication précédente, **caractérisé en ce que** le circuit de prélèvement (150) du dispositif de diagnostic (100) est relié sur une cuve du transformateur immergé (130).

12. Transformateur électrique immergé selon la revendication 10, **caractérisé en ce qu'**il comprend en outre un circuit de refroidissement et **en ce que** le circuit de prélèvement (150) dudit dispositif de diagnostic (100) est connecté :
- en dérivation sur :
∘ ledit circuit de refroidissement du diélectrique liquide dudit transformateur immergé (130), ou
∘ sur un élément dudit circuit de refroidissement ; ou
- en série dans ledit circuit de refroidissement, de sorte que l'ensemble de l'isolant liquide circulant dans ledit circuit de refroidissement circule dans ledit dispositif de diagnostic.
